# EUROPÄISCHE PATENTSCHRIFT

(11) **EP 2 604 312 B1**
(45) Veröffentlichungstag und Bekanntmachung des Hinweises auf die Patenterteilung: **01.07.2015**
(21) Anmeldenummer: 12190717.4
(22) Anmeldetag: 31.10.2012
(51) Int. Cl.: A61N 1/05

(54) **Leitungswendelanordnung und Elektrodenkatheteranordnung, insbesondere zur Kardialtherapie**
Conductive coil arrangement and electrode catheter arrangement, in particular for cardiac therapy
Agencement de conducteur spiralé et agencement de cathéter à électrode, notamment pour la thérapie cardiaque

(30) Priorität: 13.12.2011 US 201161569797 P
(43) Veröffentlichungstag der Anmeldung: 19.06.2013
(73) Patentinhaber: BIOTRONIK SE & Co. KG, 12359 Berlin (DE)
(72) Erfinder: Jadwizak, Detmar, 15537 Erkner (DE); Palm, Jochen, 15831 Mahlow (DE); Weitzig, Pierre, 10249 Berlin (DE); Fründt, Carsten, 13057 Berlin (DE); Hillebrand, Gordon, 12157 Berlin (DE); Günther, Thomas, 14552 Michendorf (DE); Steglich, Carsten, 12587 Berlin (DE); Richter, Jan Helge, 12355 Berlin (DE)
(74) Vertreter: Lindner-Vogt, Karin L.

(56) Entgegenhaltungen:
- DE-A1- 3 304 506
- US-A1- 2002 099 430
- US-A1- 2002 151 949
- US-A1- 2008 039 896

## Beschreibung

Die Erfindung betrifft eine Leitungswendelanordnung, insbesondere für Elektrodenkatheter zur Kardialtherapie, umfassend eine mehrpolige Leitungswendel mit mehreren coradial ineinander gewickelten Einzelwendeldrähten und eine Kontaktzone, in der mindestens ein Einzelwendeldraht aus der Leitungswendel mit einem Kontaktorgan zur elektrischen Kontaktierung verbindbar ist, wobei der mindestens eine zu kontaktierende Einzelwendeldraht der mehrpoligen Leitungswendel mit einer radialen Richtungskomponente aus dem Drahtverband der Leitungswendel nach außen geführt ist und die verbleibende Leitungswendel zentralaxial durch die Kontaktzone weitergeführt ist. Ferner bezieht sich die Erfindung auf eine Elektrodenkatheteranordnung, bei der eine solche Leitungswendelanordnung eingesetzt wird.

Zum Hintergrund der Erfindung ist festzuhalten, dass zur elektrischen und mechanischen Anbindung der Einzelwendeldrähte coradialer, mehrpoliger Leitungswendeln bisher bekanntermaßen ein komplexer, biegesteifer Mechanismus über eine längere Strecke verwendet wurde. Beispiel hierfür ist eine in der älteren Anmeldung mit dem Aktenzeichen US 13/205,232 gezeigte Anbindung von Einzelwendeldrähten an eine Ringelektrode eines Elektrodenkatheters bei gleichzeitiger Durchführung eines Einzelwendeldrahtes durch diese Ringelektrode.

Bekannt sind ferner Elektrodenzuleitungen in einem sogenannten Mehrlumen-Aufbau, wobei die Zuleitungen als sogenannte Seile ausgeführt sind. Diese sind an einen multipolaren Stecker, wie er beispielsweise unter der Bezeichnung "DF-4" von der Anmelderin offenkundig benutzt wird, angebunden. Die Elektrodenkatheter selbst basieren dabei auf einem mehrlumigen Schlauch, der elektrisch leitende Seile oder Wendeln in mehreren parallel zur Längsachse des Schlauchs liegenden Achsen von den Kopf- und Ringelektroden zu einem Anschlussstecker führt. Die aufeinander zulaufenden Kontaktenden der Seile oder Wendeln einerseits bzw. Kontaktelemente im Stecker andererseits haben eine axiale Ausrichtung zueinander und sind daher relativ einfach für eine entsprechende elektrische Verbindung zugänglich.

Die Anbindung von Leitungswendeln an zu kontaktierende Bauelemente erfolgt bisher vorzugsweise durch das Ablegen des jeweiligen Wendelendes oder einzelner Drähte auf Ab- oder Ansätze von Kontakthülsen, wobei die elektrische Verbindung über form- oder stoffschlüssige Verfahren, wie Crimpen, Schweißen oder Löten realisiert wird. Diese Ansätze sind beispielsweise aus den Dokumenten US 2002/0099430 A1 oder US 2008/0039896 A1 bekannt. Bekannt ist zudem das Verschweißen der Wendel in einer Bohrung, was durch die Hülse hindurch durchgeführt wird. Gemäß einer weiteren bekannten Anbindungsart - bevorzugt für nicht verschweißbare Materialkombinationen - wird das Wendelende zwischen zwei Hülsen beispielsweise durch Crimpen verbunden. Der Aufbau besteht dabei aus einer Unterhülse (starr), dazwischen liegenden Kontaktdrähten und einer Crimphülse darüber, die plastisch verformt wird, um einen Formschluss und damit einen elektrischen Kontakt zu erreichen. Dieses Anbindungsprinzip funktioniert auch bei Einzeldrähten oder Seilen. In diesem Fall liegt zwischen den Hülsen keine Wendel, sondern radial oder axial ankommende Drähte oder Seile, die aus dem Wendelverbund des Katheterkörpers kommend, passend umgelegt und/oder verformt werden. Ebenso ist es möglich, diese Einzeldrähte oder Seile zur Kontaktierung in Bohrungen oder Hülsen hineinzuführen und stoff- oder formschlüssig zu verbinden (beispielsweise durch Schweißen oder Crimpen). Die Anwendbarkeit dieser üblichen Techniken für den Anschluss von Wendeldrähten ist in vielerlei Hinsicht beschränkt, insbesondere was die Anbindung der einzelnen Wendeldrähte coradialer Mehrfachwendeln an axial verlaufende Kontaktelemente von Steckern betrifft.

Ausgehend von den geschilderten Problemen des Standes der Technik liegt der Erfindung die Aufgabe zugrunde, eine Leitungswendelanordnung der eingangs genannten Art so zu verbessern, dass eine konstruktiv einfache, universell einsetzbare Anbindung an Kontaktelemente verschiedenster Art ermöglicht wird.

Diese Aufgabe wird in einem groben Grundkonzept dadurch gelöst, dass die zentralaxial durch die Kontaktzone weitergeführte Leitungswendel ein Einzelwendeldraht ist, wobei die verbleibenden, radial herausgeführten Einzelwendeldrähte der Leitungswendel jeweils als radial versetzte und axialparallel angeordnete Kontakt-Einzelwendeln ausgebildet sind. Aufgrund dieser erfindungsgemäßen Konstruktion wird also eine coradiale Wendel in radial versetzte Einzelwendeldrähte überführt, die dann besser kontaktiert werden können. Diese Vereinzelung ist dabei für eine beliebige Anzahl von Leitern aus dem mehrpoligen Verbund der coradialen Leitungswendel möglich. Die erfindungsgemäße Leitungswendelanordnung ist in Anspruch 1 definiert.

Gemäß einer bevorzugten Ausführungsform sind die radial versetzten und axialparallel angeordneten Kontakt-Einzelwendeln peripher gleichmäßig verteilt ausgebildet. Die radial versetzten und axialparallel angeordneten Kontakt-Einzelwendeln können in bevorzugter Weise noch einen gegenüber dem Außendurchmesser der Leitungswendel kleineren Außendurchmesser aufweisen.

Die vorstehenden bevorzugten Ausführungsformen zeigen den Vorteil, dass die typischen Eigenschaften einer Wendel wie Elastizität und Unempfindlichkeit gegen Biegelastwechsel auf einem kurzen Bauraum beibehalten werden können. Durch die Reduzierung der nach außen geführten Kontakt-Einzelwendeln kann der Gesamtaußendurchmesser der Leitungswendelanordnung im Bereich der Kontaktzonen signifikant reduziert werden.

Eine Weiterbildung der Erfindung im Zusammenhang mit den nach außen geführten Einzelwendeln sieht coaxial darin eingesetzte Kontaktpins als Kontaktorgan zum elektrischen Anschluss der jeweiligen Einzelwendeldrähte vor. Da die Kontaktpins dann in axialparalleler Richtung stehen, sind diese einfach an kooperierende Kontaktelemente eines Anschlusssteckers für die Leitungswendelanordnung koppelbar.

Das oben angesprochene Grundkonzept der erfindungsgemäßen Leitungswendelanordnung kann gemäß einer alternativen Ausführungsform auch dadurch weitergebildet werden, dass die zentralaxial durch die Kontaktzone weitergeführte Leitungswendel wiederum einen Einzelwendeldraht umfasst, abweichend von der obigen Variante jedoch die verbleibenden Einzelwendeldrähte der Leitungswendel mit ihren Enden radial abstehend angeordnet und jeweils an ein Kontaktorgan angeschlossen sind. Für letztere bieten sich jeweils Kontaktblöcke oder Kontakthülsen an. Im Zusammenhang mit Kontaktblöcken können die Enden der Einzelwendeldrähte jeweils in Radialrichtung oder auch abgekröpft in Peripherrichtung in jeweilige Anschlussbohrungen der Kontaktblöcke eingesteckt und dort vorzugsweise durch Crimpen, Schweißen oder Löten festgelegt werden. Bei der Verwendung von Kontakthülsen kann eine Kontaktierung jeweils über Wandungsschlitze in den Hülsenwänden erfolgen, indem die Enden der Drähte dort in radialer Richtung eingesteckt und wiederum durch übliche Kontaktfixierungstechniken festgelegt werden. Diese Maßnahme stellte eine konstruktiv besonders einfache, zuverlässige und dabei äußerst kompakte Variante für die Vereinzelung der Wendeldrähte der coradialen Leitungswendel und deren Kontaktierung dar.

Eine weitere Fortbildung des Erfindungsgegenstandes sieht eine gleichmäßige Verteilung der Kontaktorgane wie der Einzelwendeldrähte, Kontaktblöcke oder -hülsen über den Umfang der Leitungswendelanordnung in einer gemeinsamen, also zumindest teilweise überlappenden Axialposition vor. Diese Anordnung dient wiederum der platzsparenden elektrischen Anbindung der Leitungswendel über eine kurze Axiallänge und einen kleinen Durchmesser, da die Einzelwendeldrähte im gleichen Axialebenenbereich kontaktiert werden.

Weitere bevorzugte Ausführungsformen der vorliegenden Erfindung beziehen sich auf die Anbindung der Leitungswendelanordnung an weiterführende Verbindungsorgane, wie Stecker. So ist in der Kontaktzone ein sogenannter Terminalblock vorgesehen, in oder an dem die Kontaktorgane der Einzelwendeldrähte festgelegt sind. Dieser Terminalblock stellt ein definiertes Übergangsstück von der Leitungswendelanordnung zu einem mehrpoligen Steckverbindungselement dar, wobei die im Terminalblock festgelegten Kontaktorgane der Einzelwendeldrähte entsprechend den Positionen der Kontaktpole in dem Steckverbindungselement angeordnet sind. Damit erfolgt die Kontaktierung der Einzelwendeldrähte der Leitungswendel in besonders rationeller Weise, da die Kontaktorgane entsprechend den Vorgaben der jeweiligen Steckverbindungseinheit positioniert werden können. Alternativ dazu können auch zusätzliche elektrische Kontaktelemente im Terminalblock angeordnet sein, die mit den Kontaktorganen der Einzelwendeldrähte einerseits verbunden sind. Andererseits sind diese Kontaktelemente dann mit dem jeweiligen Steckerelement in einer Elektrodenkatheteranordnung gekoppelt.

Die vorstehend erörterten, verschiedenen Konzepte einer Leitungswendelanordnung können in vorteilhafter Weise in einer Elektrodenkatheteranordnung umgesetzt werden, deren Grundkonfiguration einen langgestreckten, schlauchartigen Katheterkörper vorsieht. Mit Hilfe der erfindungsgemäßen Leitungswendelanordnung kann unter Verwendung eines entsprechenden Terminalblockes ein an sich bekannter Stecker wie ein genannter DF4/IS4-Stecker mit axial angeordneten Kontakten an einzelne, radial aus dem Verbund der Leitungswendel ankommende Wendeldrähte angeschlossen und damit elektrisch verbunden werden. Das Steckerelement kann also multipolar, vorzugsweise mindestens vierpolig, ausgelegt sein. Die coradiale Leitungswendel weist dann Einzelwendeldrähte in entsprechender Zahl, vorzugsweise vier, coradial ineinander gewickelt auf.

Nach einer besonders bevorzugten Ausführungsform sind die Leitungswendel und der Terminalblock als vorgefertigtes Modul mit dem Steckerelement endmontierbar.

Der Stecker und die ankommenden elektrischen Leitungen können damit Zugkraft übertragend und unempfindlich gegen Biegelastwechsel elektrisch und mechanisch miteinander verbunden werden. Dies erfolgt auf kleinstem Bauraum, wobei gleichzeitig ein Einzel- oder Mehrfachwendeldraht zentralaxial durch die Kontaktzone durchgeführt werden kann. Damit kann der Terminalblock gleichzeitig mit der entsprechend vorgesehenen zentralen Durchführungsöffnung zur Führung für einen Mandrin oder Führungsdraht ("guide wire"), die als Implantationshilfe bei der Implantation des Elektrodenkatheters verwendet werden, dienen.

Zusammenfassend bietet die Erfindung mit ihren bevorzugten Umsetzungsvarianten eine Vielzahl von Vorzügen gegenüber dem Stand der Technik, wie sie im Folgenden nochmals summarisch aufgelistet werden sollen:
- Durch die grundsätzliche Verwendung der Coradialwendeltechnik gegenüber Seilen wird das Problem von Schädigungen durch Kräfte und Relativbewegungen insbesondere im Anbindungsbereich an einen Stecker vermieden.
- Wendeldrähte haben gegenüber Seilen bessere Biegelastwechsel-Eigenschaften und können Zugkräfte besser übertragen. Damit können aufwendige, gewendelte Übergabestücke zur Zugentlastung der Seile vermieden werden.
- Eine Leitungswendel weist gegenüber Seilen eine bessere Stabilität gegen radial einwirkende Kräfte wie Quetschungen am Katheterkörper auf.
   Es kann eine Kontaktübergabe innerhalb eines sehr kleinen Bauraums, wie er beispielsweise durch die DF4/IS4-Steckernorm definiert ist, zwischen einem Steckelement und der Leitungswendelanordnung realisiert werden.
- Es kann eine Art Baukastensystem aufgebaut werden, indem die Leitungswendelanordnung mit einem entsprechenden Terminalblock ein Modul bildet, das mit einem entsprechenden Steckerelement als anderes Modul kombiniert werden kann.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1: eine perspektivische Ansicht einer Leitungswendelanordnung in einer ersten Ausführungsform,
- Fig. 2 bis 4: perspektivische Ansichten einer Leitungswendelanordnung mit angekoppeltem Steckerelement in einer zweiten Ausführungsform in prinzipiellen Freischneidungsgraden
- Fig. 5 und 6: perspektivische Ansichten einer Leitungswendelanordnung mit angebundenem Steckerelement in einer dritten und vierten Ausführungsform,
- Fig. 7: eine perspektivische Ansicht einer Leitungswendelanordnung mit Terminalblock,
- Fig. 8: die Anordnung gemäß Fig. 7 mit einer Abdeckkappe,
- Fig. 9: einen Axialschnitt der Anordnung gemäß Fig. 8 mit angesetztem Steckerelement,
- Fig. 10 und 11: perspektivische Ansichten der Anordnung gemäß Fig. 9 aus zwei unterschiedlichen Sichtwinkeln im Vormontagezustand, und

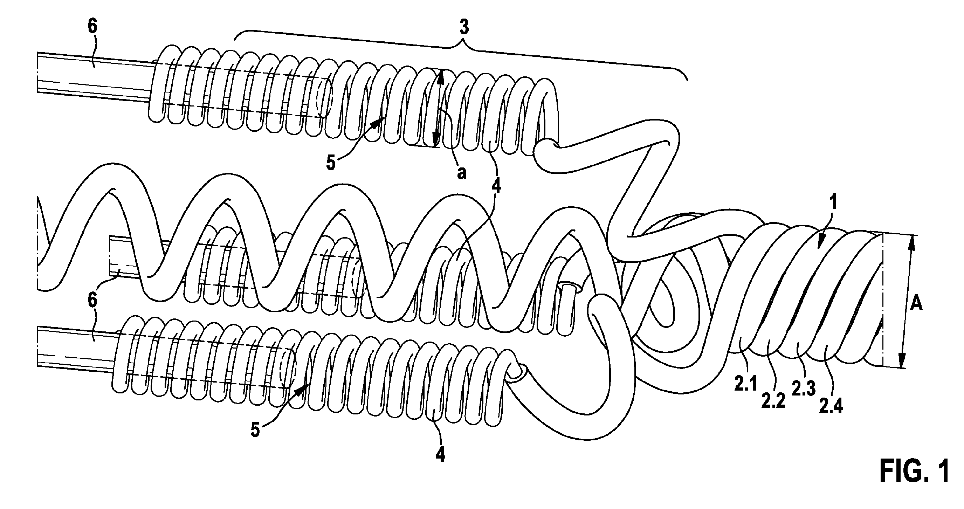

Die in Fig. 1 gezeigte Leitungswendelanordnung weist eine vierpolige, coradiale Leitungswendel 1 auf, bei der vier Einzelwendeldrähte 2.1 bis 2.4 coradial, also nach Art eines viergängigen Gewindes, auf Block ineinander gewickelt sind. Die Leitungswendel 1 endet in einer als Ganzes mit 3 bezeichneten Kontaktzone, in der die Einzelwendeldrähte 2.1 bis 2.4 an entsprechende Kontaktorgane in noch näher zu erläuternder Weise angeschlossen werden.

In der Kontaktzone 3 ist der eine Einzelwendeldraht 2.1 zentralaxial durch die Kontaktzone 3 weitergeführt, ohne dass die Ganghöhe der Wendelwicklung oder deren Durchmesser geändert wird. Die verbleibenden drei Einzelwendeldrähte 2.2 bis 2.4 werden aus dem Wendelverbund herausgelöst und in einem gleichmäßigen Versatz von 120° in Peripherrichtung zueinander schräg, also mit einer radialen Richtungskomponente und einer axialen Richtungskomponente, nach außen geführt. Die abisolierten Enden 4 der Einzelwendeldrähte 2.1 bis 2.4 sind als Kontakt-Einzelwendel mit einem gegenüber dem Außendurchmesser A der Leitungswendelanordnung deutlich kleineren Außendurchmesser a zu einer Kontaktwendel 5 gewickelt, die jeweils in axialparalleler Richtung ausgerichtet sind. Als Kontaktorgane zur elektrischen Kontaktierung der Einzelwendeldrähte 2.2 bis 2.4 sind in deren Kontaktwendeln 5 jeweils koaxiale Kontaktpins 6 eingesetzt und mechanisch fixiert. Letzteres kann durch die den Kontaktwendeln 5 innewohnende Federkraft und ein entsprechendes Übermaß des Kontaktpins 6 gegenüber dem Innendurchmesser der Kontaktwendeln 5 auf konstruktiv einfache Weise realisiert werden. Eine zusätzliche Fixierung beispielsweise mit Hilfe eines Löt- oder Schweißpunkts oder dergleichen kann aus Sicherheitsgründen vorgesehen sein. Die Kontaktpins 6 können in axialer Richtung mit den hier nicht näher dargestellten Kontaktelementen beispielsweise eines Steckerelementes verbunden und damit die Leitungswendelanordnung an ein Steckelement angeschlossen werden.

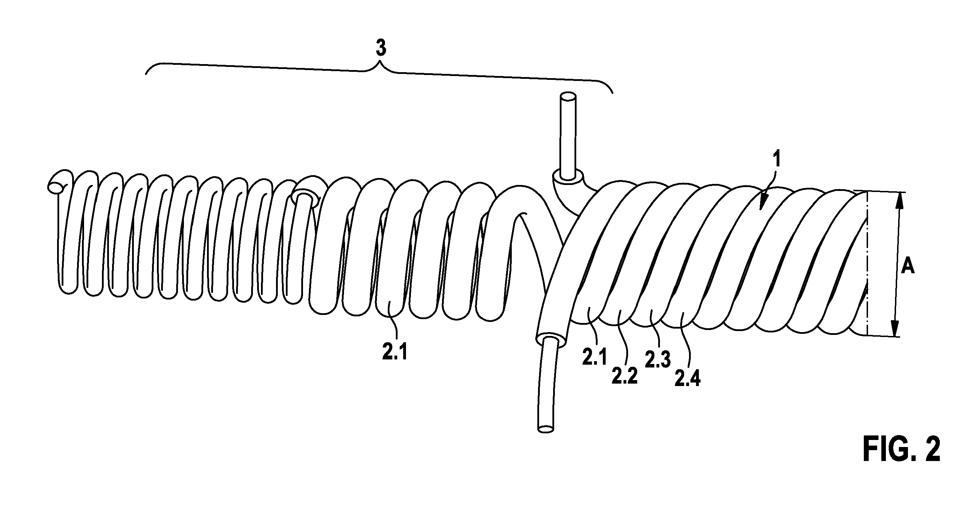

Die Fig. 2 bis 4 zeigen in verschiedenen prinzipiellen Freischneidungsgraden eine weitere Ausführungsform einer Leitungswendelanordnung, bei der die Leitungswendel 1 an ein Steckerelement 7 angebunden wird. Wie aus Fig. 2 deutlich wird, wird der eine Einzelwendeldraht 2.1 wieder koaxial weitergeführt, dabei - im Unterschied zur Ausführungsform gemäß Fig. 1 - jedoch im fortgesetzten Abschnitt auf Block gewickelt und an seinem Ende 8 abisoliert.

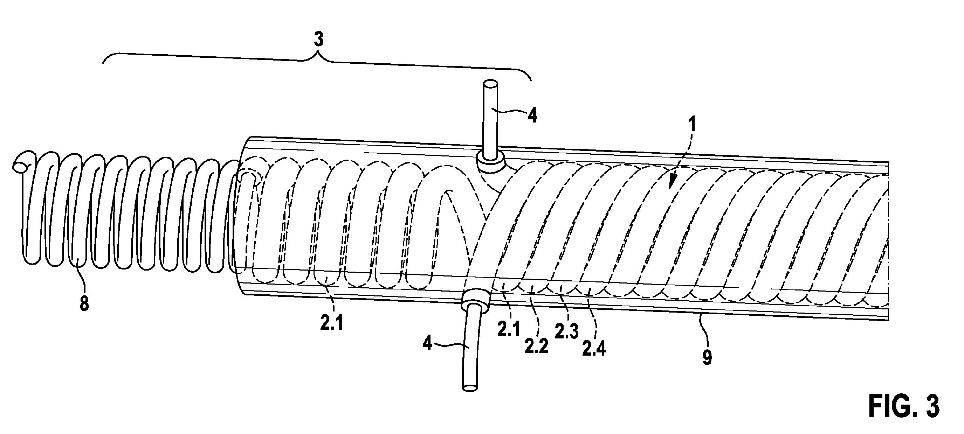

Die verbleibenden Einzelwendeldrähte 2.2 bis 2.4 werden abgelängt und in radialer Richtung abstehend von der Leitungswendel 1 weggeführt. In Fig. 2 und 3 sind aufgrund der Perspektive lediglich die beiden Enden 4 der Einzelwendeldrähte 2.3 und 2.4 erkennbar.

Die so aufbereitete Leitungswendel 1 wird anschließend mit einem Isolierschlauch 9 versehen, der vor dem abisolierten Ende 8 des zentralen Einzelwendeldrahtes 2.1 endet. Die abisolierten Enden 4 der verbleibenden Einzelwendeldrähte 2.2 bis 2.4 stehen durch den Isolierschlauch 9 in Radialrichtung nach außen ab.

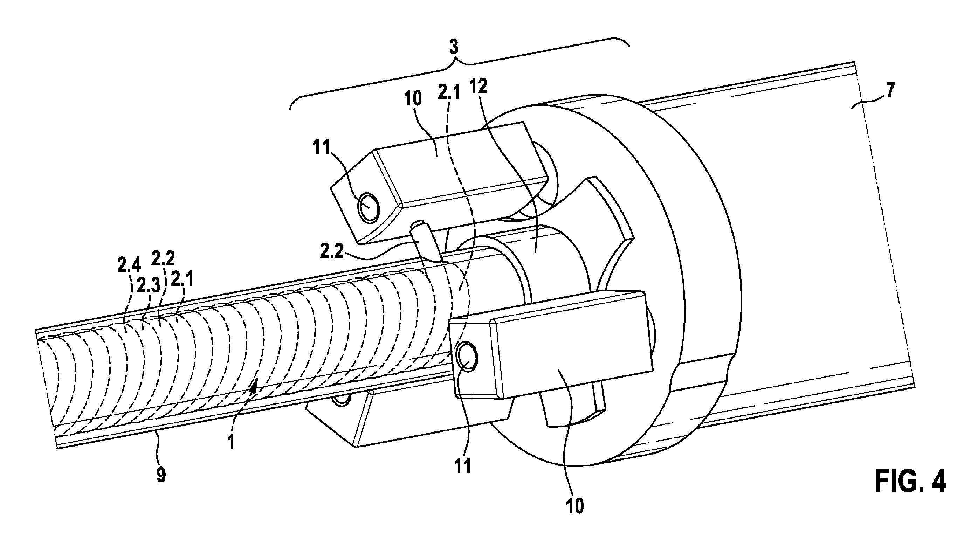

Wie aus Fig. 4 hervorgeht, werden auf diese abisolierten Enden 4 langgestrecktquaderförmige Kontaktblöcke 10 aufgesteckt und durch übliche Kontaktverbindungsmaßnahmen der Fügetechnik wie Crimpen, Schweißen oder Löten mit den Enden 4 mechanisch und elektrisch verbunden. Die Kontaktblöcke 10 weisen axialparallel verlaufende Bohrungen 11 auf, in die Kontaktstifte des Steckerelements 7 eingesetzt und mechanisch sowie elektrisch durch die üblichen, vorstehend erwähnten Fügeverfahren mechanisch und elektrisch mit den Kontaktblöcken 10 verbunden werden können.

Der zentrale Einzelwendeldraht 2.1 ist in eine entsprechende Aufnahme 12 des Steckerelementes 7 eingeführt und dort ebenfalls mechanisch und elektrisch in geeigneter Weise an einen zentralen Kontaktpol des Steckerelementes 7 angebunden.

Aufgrund der Flexibilität der Leitungswendel 1 ist der gezeigte Übergang zum Steckerelement 7 Zugkraft übertragend, unempfindlich gegen Biegelastwechsel und kompakt realisierbar.

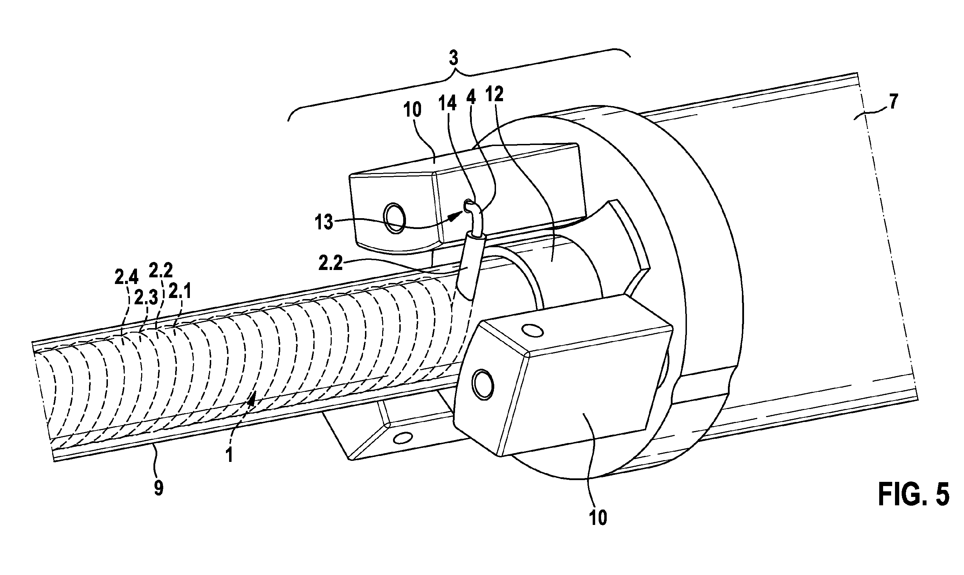

Fig. 5 zeigt eine Detailvariante der Ausführungsform gemäß Fig. 4. Die radial abstehenden Enden 4 der Einzelwendeldrähte 2.2 bis 2.4 sind hier mit einer Abkröpfung 13 in Peripherrichtung in ein entsprechendes Aufnahmeloch 14 seitlich in den Kontaktblöcken 10 eingesteckt und fixiert. Im Übrigen kann auf die Beschreibung zur Fig. 4 hinsichtlich der Kontaktblöcke 10 und deren Anbindung an das Steckerelement 7 verwiesen werden.

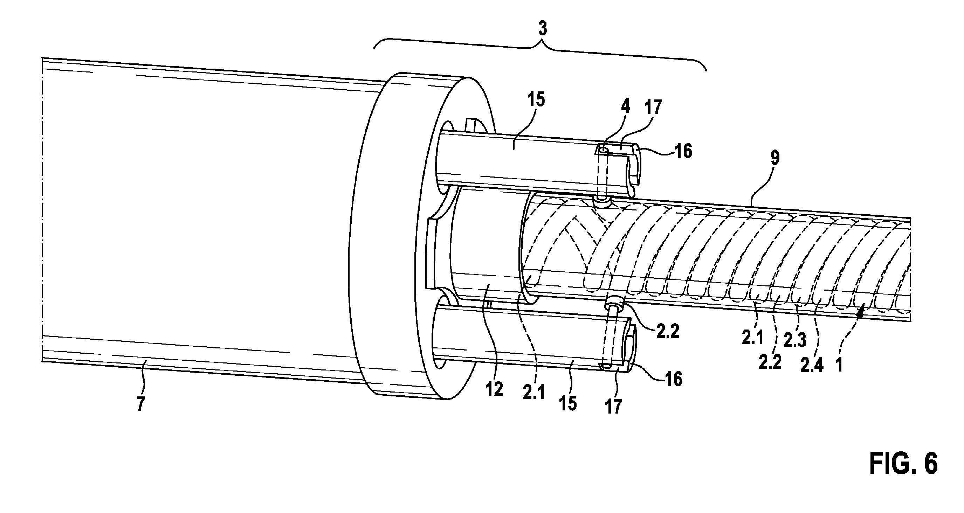

Eine weitere alternative Ausführungsform für die Anbindung eines Steckelementes 7 an eine Leitungswendel 1 ist in Fig. 6 gezeigt. Die Leitungswendel 1 weist wiederum vier Einzelwendeldrähte 2.1 bis 2.4 auf, die analog zu den in Fig. 2 und 3 gezeigten Fertigungszwischenschritten aufbereitet werden. Insoweit kann auf die dortige Beschreibung verwiesen werden.

Die radial abstehenden Enden 4 der Einzelwendeldrähte 2.2 bis 2.4 sind bei dieser Ausführungsform gemäß Fig. 6 in Kontakthülsen 15 befestigt, die wiederum axialparallel gleichmäßig in Umfangsrichtung angeordnet sind. Die Verbindung zwischen den abisolierten Enden 4 der Einzelwendeldrähte 2.2 bis 2.4 mit den Kontakthülsen 15 erfolgt dabei über in die Stirnkanten 16 der Kontakthülsen in einer Längsaxialebene eingebrachte Schlitze 17, in denen die Enden 4 wieder über die oben erwähnten Fügeverfahren festegelegt sind.

Die Kontakthülsen 15 wiederum sind mit den entsprechenden Kontaktpolen des Steckerelements 7 elektrisch und mechanisch verbunden. Gleichermaßen ist der zentrale Einzelwendeldraht 2.1 über eine Aufnahme 12 an das Steckerelement 7 angekoppelt und mit einem Zentralpol davon verbunden.

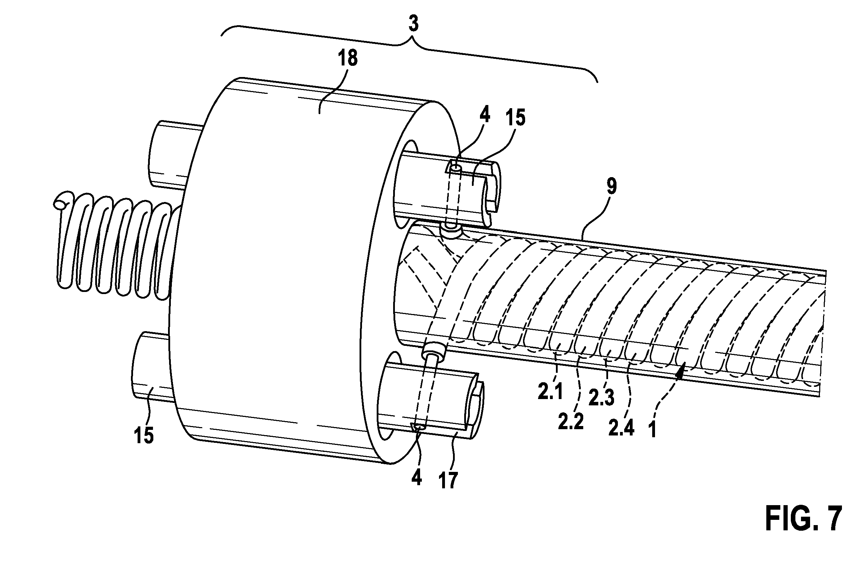

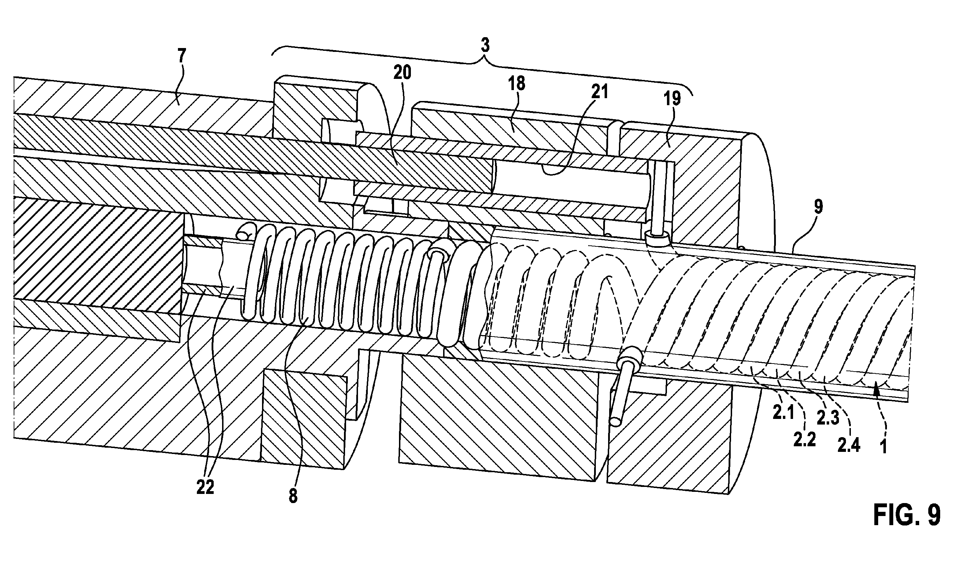

In Fig. 7 ist eine Ausführungsform einer Leitungswendelanordnung gezeigt, bei der die Kontaktorgane in Form der wie in Fig. 6 angebundenen Kontakthülsen 15 der radial nach außen weggeführten Einzelwendeldrähte 2.2 bis 2.4 und der zentralen Aufnahme 12 für den zentralen Einzelwendeldraht 2.1 in einem Terminalblock 18 festgelegt sind. Letzterer ist aus isolierendem Material gefertigt und entspricht in seiner - wie hier gezeigt - zylindrischen Formgebung im Außendurchmesser einem Steckerelement 7, mit dem der Terminalblock 18 mechanisch und mit den Kontaktorganen elektrisch an das Steckerelement angekoppelt wird, wie dies in Fig. 9 erkennbar ist und im Folgenden noch näher erläutert wird.

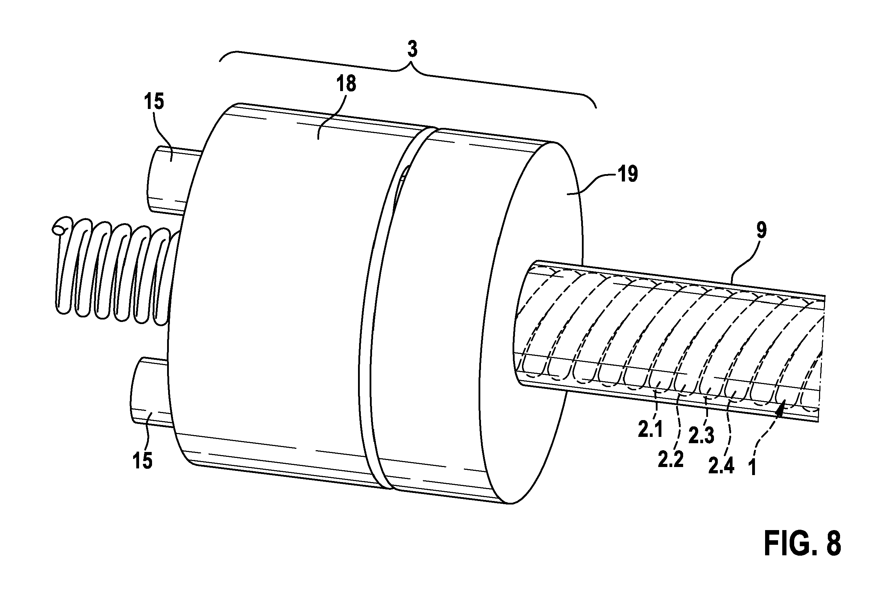

In Fig. 8 ist die Leitungswendel 1 mit Terminalblock 18 gemäß Fig. 7 gezeigt, wobei noch eine isolierende Abdeckkappe 19 den Raum umgibt, in dem die Schlitze 17 der Kontakthülsen 15 mit den abisolierten Enden 4 der Einzelwendeldrähte 2.2 bis 2.4 angeordnet sind. Damit sind diese Kontaktstellen mechanisch geschützt und elektrisch nach außen isoliert.

Die bereits erwähnte Fig. 9 zeigt die Leitungswendelanordnung mit Leitungswendel 1 und Terminalblock 18 mit Abdeckkappe 19 in einer mit dem Steckerelement 7 zusammenmontierten Konfiguration. Das Steckerelement 7 weist dabei axial mit den Kontakhülsen 15 fluchtende Kontaktstifte 20 für den jeweiligen Steckerpol auf, die jeweils in die Innenöffnung 21 der Kontakthülsen 15 eingesetzt und mechanisch sowie elektrisch damit verbunden sind. Der zentrale Einzelwendeldraht 2.1 ist mit seinem abisolierten Ende mit dem zentralen Kontaktpol 22 mechanisch und elektrisch verbunden. Insgesamt ergibt sich also eine Anbindung der vierpoligen Leitungswendel 1 an ein Steckerelement mit einem Zentralpol und drei gleichmäßig in Peripherrichtung verteilten, axialparallelen Kontaktpolen 22, von denen in Fig. 9 lediglich einer mit der zugehörigen Kontakthülse 15 erkennbar ist. Ferner ist darauf hinzuweisen, dass der Terminalblock mit einer zentral durchgehenden Führungsöffnung 24 für einen Guide wire oder einen Mandrin als Implantationshilfe versehen ist.

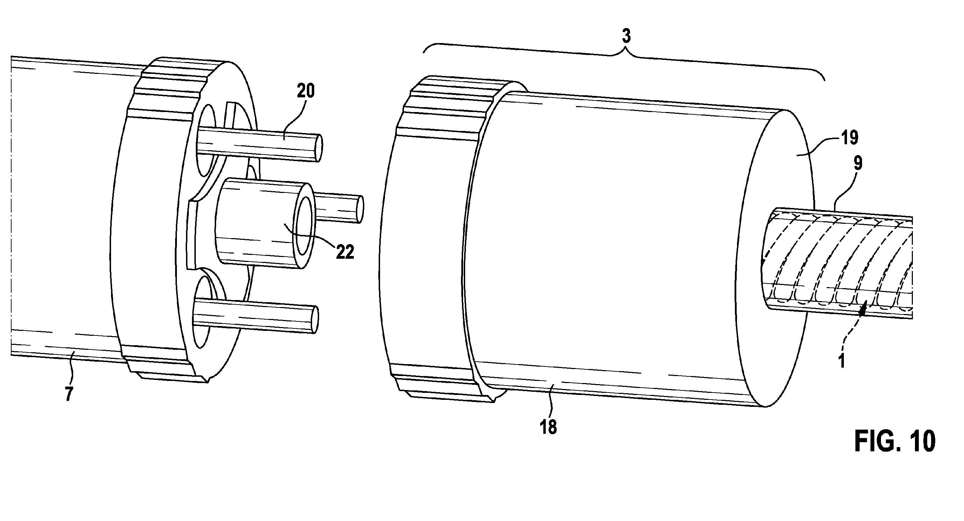

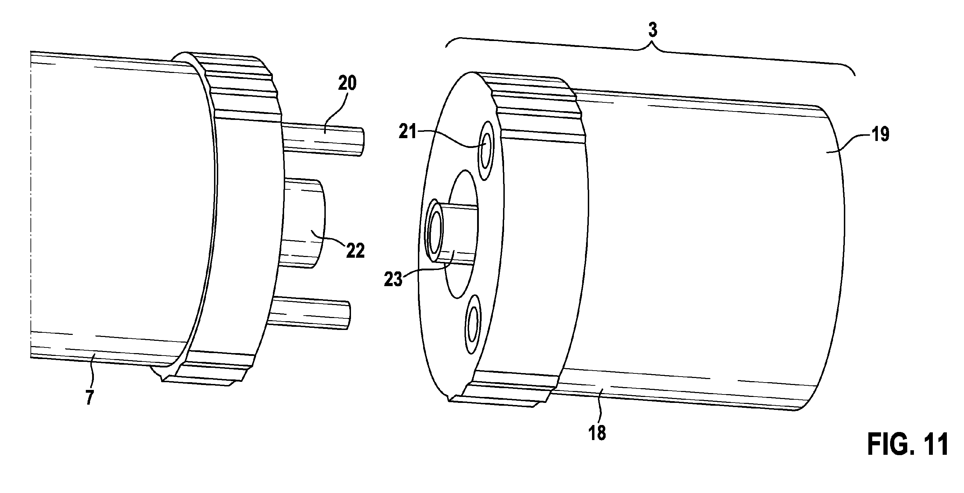

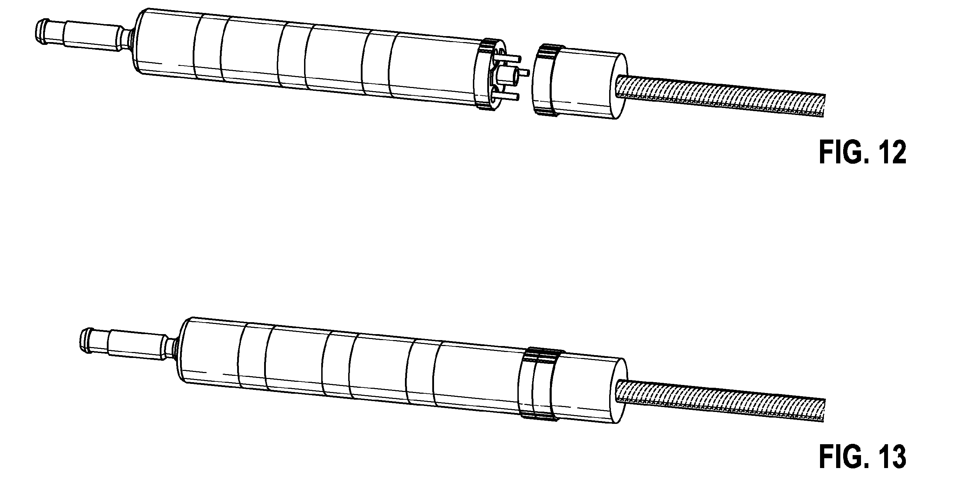

In Fig. 10 und 11 ist explizit dargestellt, dass die Leitungswendel 1 mit einem entsprechenden Isolierschlauch 9 und einem Terminalblock 18 als modulare Baugruppe genauso vormontierbar ist, wie das Steckerelement 7 mit seinen drei Kontaktstiften 20 und dem zentralen Kontaktpol 22. Die Endmontage erfolgt durch ein einfaches Zusammenstecken des Steckerelementes 7 mit dem Terminalblock 18 der Leitungswendel 1 unter mechanischer und elektrischer Verbindung zu den Kontakthülsen 15.

Bezüglich der Kontaktverbindung zwischen dem zentralen Kontaktpol 22 des Steckerelementes 7 und dem zentralen Einzelwendeldraht 2.1 ist in Fig. 11 noch eine Variante gezeigt. Letzterer ist mit einem Kontaktstift 23 an seinem Ende 8 versehen, der mit dem Kontaktpol 22 auf konstruktiv besonders einfache Weise durch Zusammenstecken verbunden werden kann.

## Patentansprüche

1. Leitungswendelanordnung, insbesondere für Elektrodenkatheter zur Kardialtherapie, umfassend
- eine mehrpolige Leitungswendel (1) mit mehreren coradial ineinander gewickelten Einzelwendeldrähten (2.1 - 2.4), und
- eine Kontaktzone (3), in der mindestens ein Einzelwendeldraht (2.1 - 2.4) aus der Leitungswendel mit einem Kontaktorgan (6) elektrisch verbunden ist,
wobei
- der mindestens eine zu kontaktierende Einzelwendeldraht (2.1 - 2.4) mit einer radialen Richtungskomponente aus dem Drahtverbund der Leitungswendel (1) nach außen geführt ist, und
- die verbleibende Leitungswendel (2.1) zentralaxial durch die Kontaktzone (3) weitergeführt ist,
**dadurch gekennzeichnet, dass** die zentralaxial durch die Kontaktzone (3) weitergeführte Leitungswendel einen Einzelwendeldraht (2.1) umfasst und die verbleibenden radial herausgeführten Einzelwendeldrähte (2.2 - 2.4) der Leitungswendel (1) in der Kontaktzone (3) jeweils als radial versetzte und axialparallel angeordnete, vorzugsweise peripher gleichmäßig verteilte Kontakt-Einzelwendeln (2.2 - 2.4) ausgebildet sind.

2. Leitungswendelanordnung nach Anspruch 1, **dadurch gekennzeichnet, dass** die radial versetzte und axialparallel angeordneten Einzelwendeln (2.2 - 2.4) einen gegenüber dem Außendurchmesser (A) der Leitungswendel (1) kleineren Außendurchmesser (a) aufweisen.

3. Leitungswendelanordnung nach Anspruch 1 oder 2, **dadurch gekennzeichnet, dass** in die radial versetzte und axialparallel angeordneten Einzelwendeln (1) koaxiale Kontaktpins (6) als Kontaktorgan zu ihrem elektrischen Anschluss eingesetzt sind.

4. Leitungswcndelanordnung nach einem der vorgenannten Ansprüche, **dadurch gekennzeichnet, dass** die Kontaktorgane (6, 10, 15) gleichmäßig über den Umfang der Leitungswendelanordnung verteilt und in einer gemeinsamen Axialposition angeordnet sind.

5. Leitungswendelanordnung nach einem der vorgenannten Ansprüche, **dadurch gekennzeichnet, dass** in der Kontaktzone (3) ein Terminalblock (18) vorgesehen ist, in oder an dem die Kontaktorgane (6, 10, 15) der Einzelwendeldrähte (2.1 - 2.4) festgelegt sind.

6. Leitungswendelanordnung nach Anspruch 5, **dadurch gekennzeichnet, dass** im Terminalblock (18) elektrische Kontaktelemente (20) angeordnet sind, die einerseits mit den Kontaktorganen (6, 10, 15) der Einzelwendeldrähte (2.1 - 2.4) verbunden oder durch diese gebildet sind, sowie andererseits mit einem Steckerelement (7) in einer Elektrodenkatheteranordnung gekoppelt sind.

7. Elektrodenkatheteranordnung, insbesondere zur Kardialtherapie, umfassend
- einen langgestreckten, schlauchartigen Katheterkörper (9),
**gekennzeichnet durch**
- eine Leitungswendelanordnung nach einem der Ansprüche 1 bis 6, und
- ein mit dem Terminalblock (18) gekoppeltes Steckerelement (7) nach Anspruch 11.

8. Elektrodenkatheteranordnung nach Anspruch 7, **dadurch gekennzeichnet, dass** das Steckerelement (7) multipolar, vorzugsweise mindestens vierpolig ausgelegt ist und die coradiale Leitungswendel (1) Einzelwendeldrähte (2.1 - 2.4) in entsprechender Zahl, vorzugsweise vier, coradial ineinander gewickelt aufweist.

9. Elektrodenkatheteranordnung nach Anspruch 7 oder 8, **dadurch gekennzeichnet, dass** die Leitungswendel (1) und der Terminalblock (18) als vorgefertigtes Modul mit dem Steckerelement (7) endmontiert sind.

10. Elektrodenkatheteranordnung nach einem der Ansprüche 7 bis 9, **dadurch gekennzeichnet, dass** der Terminalblock (18) mit einer Führungsöffnung (24) für eine Implantationshilfe im Bereich der zentralaxial durchgeführten Leitungswendel (1) versehen ist.

## Claims

1. A conductive coil arrangement, in particular for electrode catheters for cardiac therapy, comprising
- a multipolar conductive coil (1) having a plurality of coradially wound into one another, individual coil wires (2.1 - 2.4), and
- a contact zone (3) in which at least one individual coil wire (2.1 - 2.4) from the conductive coil is electrically connected to a contact element (6),
wherein
- the at least one contacting individual coil wire (2.1 - 2.4) is routed outwardly out of the wire interconnection of the conductive coil (1) with a radial direction component, and
- the remainder of the conductive coil (2.1) is routed further centrally axially through the contact zone (3),
**characterized in that** the conductive coil, which is routed further centrally axially through the contact zone (3), comprises an individual coil wire (2.1), and the remaining radially outwardly extending individual coil wires (2.2 - 2.4) of the conductive coil (1) in the contact zone (3) are each in the form of individual contact coils (2.2 - 2.4) which are radially offset and disposed axially parallel, preferably being distributed equally around the periphery.

2. The conductive coil arrangement according to claim 1, **characterized in that** the radially offset and axially parallel disposed individual coils (2.2 - 2.4) have a smaller outer diameter (a) relative to the outer diameter (A) of the conductive coil (1).

3. The conductive coil arrangement according to claim 1 or 2, **characterized in that** coaxial contact pins (6) are inserted into the radially offset and axially parallel disposed individual coils (1), as the contact element for the electrical connection thereof.

4. The conductive coil arrangement according to any one of the preceding claims, **characterized in that** the contact elements (6, 10, 15) are distributed equally around the circumference of the conductive coil arrangement, and are disposed in a common axial position.

5. The conductive coil arrangement according to any one of the preceding claims, **characterized in that** a terminal block (18) is provided in the contact zone (3), in or at which the contact elements (6, 10, 15) of the individual coil wires (2.1 - 2.4) are secured.

6. The conductive coil arrangement according to claim 5, **characterized in that** electrical contact elements (20) are disposed in the terminal block (18), which are connected to the contact elements (6, 10, 15) of the individual coil wires (2.1 - 2.4) or are formed thereby, and to a plug element (7) in an electrode catheter arrangement.

7. An electrode catheter arrangement, in particular for cardiac therapy, comprising
- an elongated, tube-type catheter body (9),
**characterized by**
- a conductive coil arrangement according to any one of claims 1 to 6, and
- a plug element (7) according to claim 11, which is coupled to the terminal block (18).

8. The electrode catheter arrangement according to claim 7, **characterized in that** the plug element (7) is multipolar, preferably at least four-poled, and the coradial conductive coil (1) comprises individual coil wires (2.1 - 2.4) in an appropriate number, preferably four, wound into one another in a coradial manner.

9. The electrode catheter arrangement according to claim 7 or 8, **characterized in that** the conductive coil (1) and the terminal block (18) undergo final assembly as a prefabricated module with the plug element (7).

10. The electrode catheter arrangement according to any one of claims 7 to 9, **characterized in that** the terminal block (18) is provided with a guide opening (24) for an implantation aid in the region of the centrally axially routed conductive coil (1).

## Revendications

1. Agencement de conducteur spiralé, en particulier pour des cathéters à électrode servant à la thérapie cardiaque, comprenant
- un conducteur spiralé multipolaire (1) avec plusieurs fils spiralés individuels (2.1 - 2.4) enroulés co-radialement les uns dans les autres, et
- une zone de contact (3) dans laquelle au moins un fil spiralé individuel (2.1 - 2.4) du conducteur spiralé est électriquement relié à un organe de contact (6),
dans lequel
- l'au moins un fil spiralé individuel (2.1 - 2.4) à connecter est guidé vers l'extérieur avec une composante directionnelle radiale, hors de l'ensemble de fils du conducteur spiralé (1), et
- le conducteur spiralé (2.1) restant est guidé plus loin à travers la zone de contact (3) de façon centrée axialement,
**caractérisé en ce que** le conducteur spiralé guidé plus loin à travers la zone de contact (3) de façon centrée axialement comprend un fil spiralé individuel (2.1), et les autres fils spiralés individuels (2.2 - 2.4) du conducteur spiralé (1) guidés radialement vers l'extérieur sont, dans la zone de contact (3), respectivement conçus comme des fils de contact individuels (2.2 - 2.4) décalés radialement et agencés de façon axialement parallèle, en étant de préférence répartis de façon régulière sur le pourtour.

2. Agencement de conducteur spiralé selon la revendication 1, **caractérisé en ce que** les fils de contact individuels (2.2 - 2.4) décalés radialement et agencés de façon axialement parallèle présentent un diamètre extérieur (a) inférieur au diamètre extérieur (A) du conducteur spiralé (1).

3. Agencement de conducteur spiralé selon la revendication 1 ou 2, **caractérisé en ce que** des broches de contact (6) coaxiales sont introduites dans les fils spiralés individuels (1) décalés radialement et agencés de façon axialement parallèle, en tant qu'organe de contact pour leur raccordement électrique.

4. Agencement de conducteur spiralé selon l'une des revendications précédentes, **caractérisé en ce que** les organes de contact (6, 10, 15) sont répartis de façon régulière sur le pourtour de l'agencement de conducteur spiralé et agencés dans une position axiale commune.

5. Agencement de conducteur spiralé selon l'une des revendications précédentes, **caractérisé en ce que** dans la zone de contact (3), il est prévu un bloc de jonction (18) dans lequel ou sur lequel sont fixés les organes de contact (6, 10, 15) des fils spiralés individuels (2.1 - 2.4).

6. Agencement de conducteur spiralé selon la revendication 5, **caractérisé en ce que** des éléments de contact électriques (20) sont agencés dans le bloc de jonction (18), lesquels sont d'une part reliés aux organes de contact (6, 10, 15) des fils spiralés individuels (2.1 - 2.4) ou formés par ceux-ci, et d'autre part couplés à un élément de connecteur (7) dans un agencement de cathéter à électrode.

7. Agencement de cathéter à électrode, en particulier pour la thérapie cardiaque, comprenant
- un corps de cathéter (9) tubulaire allongé,
**caractérisé par**
- un agencement de conducteur spiralé selon l'une des revendications 1 à 6, et
- un élément de connecteur (7) selon la revendication 11, couplé au bloc de jonction (18).

8. Agencement de cathéter à électrode selon la revendication 7, **caractérisé en ce que** l'élément de connecteur (7) est conçu multipolaire, de préférence avec au moins quatre pôles, et le conducteur spiralé (1) co-radial comporte un nombre correspondant de fils spiralés individuels (2.1 - 2.4), de préférence quatre, enroulés co-radialement les uns dans les autres.

9. Agencement de cathéter à électrode selon la revendication 7 ou 8, **caractérisé en ce que** le conducteur spiralé (1) et le bloc de jonction (18) sont assemblés en tant que module préfabriqué avec l'élément de connecteur (7).

10. Agencement de cathéter à électrode selon l'une des revendications 7 à 9, **caractérisé en ce que** le bloc de jonction (18) est pourvu d'une ouverture de guidage (24) pour faciliter l'implantation dans la région du conducteur spiralé (1) guidé de façon axialement centrée.
